# EUROPEAN PATENT APPLICATION

(11) **EP 4 159 721 A1**
(43) Date of publication of application: **05.04.2023**
(21) Application number: 21306368.8
(22) Date of filing: 30.09.2021
(51) Int. Cl.: C07D 215/18, C07D 215/22, C07D 215/227, C07D 215/233, C07D 471/04, A61K 31/14, A61P 31/14, A61K 31/4704, A61K 31/437, A61K 31/4375

(54) **CONDENSED 3,3-DIFLUORO-PIPERIDINE DERIVATIVES AS INHIBITORS OF CORONAVIRUSES 3-CHYMOTRYPSIN-LIKE PROTEASE**

(71) Applicant: Qubit Pharmaceuticals, 75014 Paris (FR)
(72) Inventor: The designation of the inventor has not yet been filed
(74) Representative: A.P.I. Conseil

(57) **Abstract**

The invention relates to compounds of formula I :

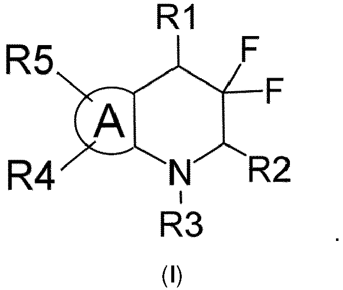

More particularly the invention relates to such compounds as inhibitors of 3CLpro protease. Accordingly, invention relates to a pharmaceutical composition comprising said compounds, and also to said compound for their use as a medicament, more particularly in the treatment or the prophylaxis of an infection to coronavirus and more particularly to SARS-CoV-2.

## Description

### FIELD OF THE INVENTION

Invention belongs to the field of medicine, more specifically to the field of treatment of coronavirus infections. More particularly invention relates to novel inhibitors of the coronavirus 3-chymotrypsin-like protease. Even more particularly, invention relates to the use of these compounds in the prophylaxis and the treatment of coronavirus-related condition, as acute pneumopathy, in particular related to COVID-19.

### BACKGROUND OF THE INVENTION

In a context of overcrowding, deterioration of biodiversity and globalization, human activity has made humans closer to wild animals leading to the expectation of the near emergence of a new disease for human species. Coronaviruses have been discovered and are responsible for zoonoses that have emerged during the twentieth century. Their reservoir is thought to be warm blood vertebrates as bats, but their host target can change to humans because of mutations which eventually allow interhuman transmission of the disease. First alerts for coronavirus pandemic occurred in the 21^{st} century, with between 2002 and 2004 the first pandemic known as SRAS (due to SARS-CoV coronavirus), and during the years 2012/2014 with the emergence of MERS (Middle East respiratory syndrome, due to MERS-CoV coronavirus). Year 2019 has seen the emergence of COVID-19 caused by SARS-CoV-2 which spread in 2019 from China to the entire world and unlike SRAS has not yet extinguished by itself and gave through several waves of pandemics in relation to the appearance of new variants of the virus.

Most infected individuals are asymptomatic or show symptoms similar to cold, but COVID-19 can turn out to be a severe pneumopathy that necessitates hospitalization, intensive care up to ventilatory support. Because of its high rate of infection, COVID-19 already caused the death of millions of people, overwhelmed health services and had serious consequences to the world economy.

Several vaccines have been developed and seems to be useful to contain the spread of the disease. Nevertheless, vaccines are expensive and not all countries have access to the most efficient ones. Further, as being essentially of prophylactic use, face to the risk of emergence of variants resistant to vaccines and because some people cannot be injected or will not show a vaccinal response, there is a need for alternative solutions to vaccines for a therapeutic or even prophylactic solution to the disease.

Two mixes of two monoclonal antibodies (Casirivimab/lmdevimab and Bamlanivimab/ Etesevimab) are currently subjected to emergency use authorization in the USA and in Europe for people at risk of developing a severe COVID-19. Casirivimab/lmdevimab is approved in Japan for the treatment of mild to moderate COVID-19. Anyway, because of their costs, dosing time frame and route of administration, these treatments remain unaffordable and barely adapted to outpatients or patients at an advance stage of the disease.

Research for small molecules as drug candidates against COVID-19 is actively ongoing. Several repurposed drugs are currently under trial (Viveiros Rosa, 2020). Some of them, like chloroquine or hydroxychloroquine, have already proved disappointing (Ryan et al., 2021).

As the other coronaviruses, SARS-CoV-2 is positive-stranded RNA virus, which shares 89% homology with the other SARS-CoVs and a similar organization of its genome. Its genome encodes a polypeptide chain which has to be cleaved to generate functional viral proteins and reconstitutes virions. Two proteases are responsible for this proteolytic processing. The first one is called main protease (Mpro), also called 3C-like protease (3CLpro) and the other is Papain like protease (PLpro). 3CLpro is responsible for the generation of 11 out of 16 of the viral proteins thereby produced. This pivotal role in infection cycle makes 3CLpro a valuable target for the development of novel treatments against SARS-CoVs and more particularly SARS-CoV-2 (Mody et al., 2021 ; Anand et al., 2003). Several drugs have been tested for their ability to inhibit SARS-CoV-2's 3CLpro (Mody et al., 2021), amongst which proteases inhibitors used for other viral infections or repurposed drugs as ivermectin which effectiveness *in vivo* treating or preventing the disease has still to be demonstrated (Popp et al., 2021). Other candidates are peptidomimetics like PF-07321332, a reversible covalent inhibitor currently under phase I clinical trial (<https://cen.acs.org/acs-news/acs-meeting-news/Pfizer-unveils-oral-SARS-CoV/99/i13>).

There is an urgent need for the development of several alternatives in the class of 3CLpro inhibitors, which constitutes a promising way in the search for a treatment against infection to coronavirus and, more particularly, Sars-CoV-2.

Thanks to a proprietary simulation platform of computational chemistry, inventors have been able to identify a novel family of molecules which are potent inhibitors of SARS-CoV-2 3CLpro and therefore constitute valuable candidates in the treatment and prophylaxis of COVID-19 and other coronavirus infections.

Invention therefore aims to provide novel inhibitors of 3CLpro of SARS-CoV-2. Indeed, inventors have identified that compounds of a specific chemical structural core that are able to bind and inhibit protease activity of 3CLpro of SARS-Cov2. Also because of their ability to disrupt viral multiplication cycle, these compounds are useful for treating or prevent SARS-Cov2 infection or a condition or symptom resulting form said infection. These compounds are therefore also particularly useful in preventing worsening of COVID-19 in a subject infected by SARS-CoV-2. Though particularly adapted to 3CLpro of SARS-CoV-2 these compounds constitute valuable candidate compounds for treating infection to other coronaviruses.

### SUMMARY OF THE INVENTION

Accordingly, invention relates to a compound of general formula I : 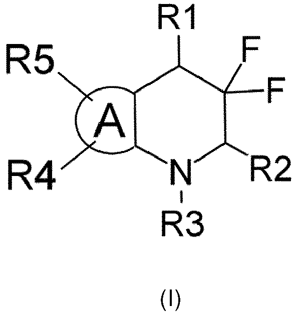 in any enantiomeric, diastereomeric form, or any salt and/or solvate thereof, wherein :
- R₁ and R₂ are each independently from the other selected from : a hydrogen atom, (C₁-C₆) alkyl, OH, OR₆, SR₆ and an oxo group,
- R3 is selected from a hydrogen atom or a (C₁-C₆) alkyl,
- ring A is selected from a 5-membered aromatic ring, a 5-membered heteroaromatic ring, a 6 membered aromatic cycle or a 6 membered heteroaromatic ring,
- R₄ is selected from SO₂R₇, SONHR₇, OR₇, SOR₇, SR₇, (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
- R₅ is selected from H or a halogen atom,
- R₆ is a (C₁-C₆) alkyl, R₇ is selected from (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
with the proviso that at least one of R1 or R2 is different from an hydrogen atom.

More particularly, the compound of formula I wherein R₁ and R₂ are each independently from the other selected from a hydrogen atom, OH, (C₁-C₆) alkyl, and an oxo group constitutes a particular embodiment.

Also in another particular embodiment, in the compound of formula I ring A is selected from a 6 membered aromatic cycle or a 6 membered heteroaromatic ring. In a further particular embodiment, in said compound, R₄ is selected from SR7, (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl.

In a particular embodiment, the above compound more particularly relates to a compound of formula Ia : 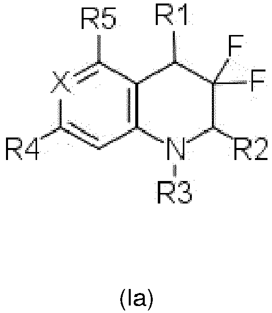 wherein X is an atom selected from C and N, in any enantiomeric, diastereomeric form, or any salt and/or solvate thereof.

In another particular embodiment, the above compound more particularly relates to a compound of formula Ib: 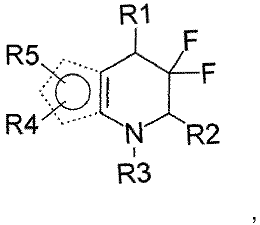 in any enantiomeric, diastereomeric form, or any salt and/or solvate thereof.

The following compounds constitute particular embodiment of the above described families of compounds : 3,3-difluoro-(R)-4-hydroxy-7-(methylsulfanyl)-1,2,3,4-tetrahydroquinolinone ; 3,3-difluoro-(S)-4-hydroxy-7-(methylsulfanyl)-1,2,3,4-tetrahydroquinolinone ; 3,3-difluoro-7-(methylsulfanyl)-1,2,3,4-tetrahydro-1,6-naphthyridin-4-(R)-ol ; 3,3-difluoro-7-(methylsulfanyl)-1,2,3,4-tetrahydro-1,6-naphthyridin-4-(S)-ol S ; 3,3-difluoro-(R)-4-hydroxy-7-(methylsulfanyl)-1,2,3,4-tetrahydroquinolin-2-one ; 3,3-difluoro-(R)-4-hydroxy-7-(methylsulfanyl)-1,2,3,4-tetrahydroquinolin-2-one ; 3,3-difluoro-(R)-4-hydroxy-7-(methylsulfanyl)-1,2,3,4-tetrahydro-1,6-naphthyridin-2-one ; 3,3-difluoro-(S)-4-hydroxy-7-(methylsulfanyl)-1,2,3,4-tetrahydro-1,6-naphthyridin-2-one ; 3,3-difluoro-(S)-4-methyl-7-(methylsulfanyl)-1,2,3,4-tetrahydroquinolin-2-one ; 3,3-difluoro-(R)-4-methyl-7-(methylsulfanyl)-1,2,3,4-tetrahydroquinolin-2-one ; 3,3-difluoro-(S)-4-methyl-7-(methylsulfanyl)-1,2,3,4-tetrahydroquinoline ; 3,3-difluoro-(R)-4-methyl-7-(methylsulfanyl)-1,2,3,4-tetrahydroquinoline ; 3,3-difluoro-7-(methylsulfanyl)-1,2,3,4-tetrahydro-1,6-naphthyridine-2,4-dione ; 3,3-difluoro-7-(methylsulfanyl)-1,2,3,4-tetrahydroquinoline-2,4-dione ; 3,3-difluoro-(R)-4-hydroxy-7-(cyclopropylsulfanyl)-1,2,3,4-tetrahydroquinoline-2-one ; 3,3-difluoro-(S)-4-hydroxy-7-(cyclopropylsulfanyl)-1,2,3,4-tetrahydroquinoline-2-one ; 3,3,5-trifluoro-(R)-4-hydroxy-7-(methylylsulfanyl)-1,2,3,4-tetrahydroquinoline-2-one ; 3,3,5-trifluoro-(S)-4-hydroxy-7-(methylylsulfanyl)-1,2,3,4-tetrahydroquinoline-2-one ; 5,5-difluoro-(R)-4-hydroxy-1,3-dimethyl-1H,4H,5H,6H,7H-pyrazolo[3,4-b]pyridin-6-one ; 5,5-difluoro-(S)-4-hydroxy-1,3-dimethyl-1H,4H,5H,6H,7H-pyrazolo[3,4-b]pyridin-6-one ; 5,5-difluoro-(R)-4-hydroxy-3-methyl-1H,4H,5H,6H,7H-pyrazolo[3,4-b]pyridin-6-one ; 5,5-difluoro-(S)-4-hydroxy-3-methyl-1H,4H,5H,6H,7H-pyrazolo[3,4-b]pyridin-6-one ; 3,5,5-trifluoro-(R)-4-hydroxy-1-methyl-1H,4H,5H,6H,7H-pyrazolo[3,4-b]pyridin-6-one ; 3,5,5-trifluoro-(S)-4-hydroxy-1-methyl-1H,4H,5H,6H,7H-pyrazolo[3,4-b]pyridin-6-one; 5,5-difluoro-1,3-dimethyl-1H,4H,5H,6H,7H-pyrazolo[3,4-b]pyridine-4,6-dione.

As shown by the inventors, a compound showing a core structure as described above constitutes a particularly effective inhibitor of 3CLpro and is capable of *in vitro* inhibiting a viral 3-chymotrypsin-like protease (3CLpro), for example a 3CLpro of a coronavirus, for example of MERS-CoV, of a SARS-CoV, for example, a 3CLpro of SARS-CoV-1 or a 3CLpro of SARS-CoV-2.

In a particular embodiment, said compound inhibitor of 3CLpro is a covalent binder of a viral 3-chymotrypsin-like protease (3CLpro), for example a 3CLpro of a coronavirus, for example of MERS-CoV, a SARS-CoV, for example, a 3CLpro of SARS-CoV-1 or a 3CLpro of SARS-CoV-2. Covalent bonding in the field of drug discovery constitutes a property of a particular interest.

In another particular embodiment, said compound inhibitor of 3CLpro is a non-covalent binder of a viral 3-chemotrypsin-like protease (3CLpro), for example a 3CLpro of a coronavirus, for example of MERS-CoV, a SARS-CoV, for example, a 3CLpro of SARS-CoV-1 or a 3CLpro of SARS-CoV-2.

Biological activity of the compound of a formula as described above, makes it of particular interest in the field of medicine, also an object of the invention is a pharmaceutical composition comprising a compound as described above.

Also, accordingly, a particular object of the invention relates to compound of a formula as described above for use as a medicament.

Compound of the invention as described above is found by the inventor as effective in inhibiting the activity of coronavirus 3CLpro, which is a key enzyme in coronavirus viral cycle. Accordingly, a more particular object of the invention relates to a compound of a formula as described above for use in the prophylaxis, treatment or combination thereof of an infection to a coronavirus in a subject in need thereof.

Also, accordingly, another particular object of the invention relates to a compound of a formula as described above for use in the prophylaxis, treatment or combination thereof, of a condition or symptom caused by a coronavirus.

In a particular embodiment, said coronavirus is SARS-CoV-2.

### FIGURE LEGENDS

**Figure 1** represents synthesis steps of compounds of the invention.
**Figure 2** shows electrospray ionisation mass spectra obtained for 3CLpro alone (A) or 3CLpro after incubation with compound 14 of the invention (B).

### DETAILED DESCRIPTION AND ADDITIONAL EMBODIMENTS

### Definitions

The term "halogen" as used in the present invention refers to an atom of fluorine, bromine, chlorine or iodine. Advantageously, it is selected between fluorine, chlorine and bromine, even more advantageously it is an atom of fluorine.

The term "(C₁-C₆)alkyl" as used in the present invention refers to a saturated, linear or branched hydrocarbon chain comprising from 1 to 6 carbon atoms, including, but not limited to, methyl, ethyl, n-propyl, isopropyl, n-butyl, isobutyl, sec-butyl, *tert*-butyl, n-pentyl, n-hexyl and the like.

The term "cycloalkyl" as used in the present invention refers to a saturated or unsaturated hydrocarbon ring comprising from 3 to 10, advantageously from 5 to 7, carbon atoms including, but not limited to, cyclopropyl, cyclobutyl, cyclopentyl, cyclohexyl, cycloheptyl and the like. A "cycloalkyl" can also refers to a polycycle, comprising fused, bridged or spiro saturated rings, preferably fused rings, advantageously comprising 3 to 10, notably 3 to 6, atoms in each ring,

The term "heterocycloalkyl" as used in the present invention refers to a cycloalkyl as stated above, in which the atoms of the ring(s) comprise one or more, advantageously 1 to 3, heteroatoms selected from O, S and N, preferably O and N, the remainder being carbon atoms.

A "heterocycloalkyl" is more particularly a 3-, 4-, 5- or 6-membered, even more particularly a 5- or 6-membered saturated monocyclic heterocycle such as an aziridine, an azetidine, a pyrrolidine, a tetrahydrofuran, a 1,3-dioxolane, a tetrahydrothiophene, a thiazolidine, an isothiazolidine, an oxazolidine, an isoxazolidine, an imidazolidine, a pyrazolidine, a triazolidine, a piperidine, a piperazine, a 1,4-dioxane, a morpholine or a thiomorpholine.

An unsaturated heterocycloalkyl is more particularly an unsaturated monocyclic or bicyclic heterocycloalkyl, each cycle comprising 5 or 6 members, such as 1*H*-azirine, a pyrroline, a dihydrofuran, a 1,3-dioxolene, a dihydrothiophene, a thiazoline, an isothiazoline, an oxazoline, an isoxazoline, an imidazoline, a pyrazoline, a triazoline, a dihydropyridine, a tetrahydropyridine, a dihydropyrimidine, a tetrahydropyrimidine, a dihydropyridazine, a tetrahydropyridazine, a dihydropyrazine, a tetrahydropyrazine, a dihydrotriazine, a tetrahydrotriazine, a 1,4-dioxene, an indoline, a 2,3-dihydrobenzofuran (coumaran), a 2,3-dihydrobenzothiophene, a 1,3-benzodioxole, a 1,3-benzoxathiole, a benzoxazoline, a benzothiazoline, a benzimidazoline, a chromane or a chromene.

The term "cycloalkyl-(C₁-C₆)alkyl" as used herein refers to any cycloalkyl as defined above, which is bound to the molecule by means of a (C1-C6)-alkyl as defined above.

The term "heterocycloalkyl-(C1-C6)alkyl" as used herein refers to a heterocycle group as defined above, which is bound to the molecule by means of a (C1-C6)-alkyl group as defined above.

The term "aryl", as used herein, refers to an aromatic hydrocarbon group comprising preferably 6 to 14 carbon atoms and comprising one or more fused rings, such as, for example, a phenyl, naphthyl or anthracenyl group. Advantageously, aryl is a phenyl group.

The term "heteroaryl" refers to an aromatic hydrocarbon group as defined above wherein the atoms of the ring(s) comprise one or more, advantageously 1 to 3, heteroatoms selected from O, S and N, preferably O and N, the remainder being carbon atoms. It can be more particularly an aromatic monocyclic, bicyclic or tricyclic heterocycle, each cycle comprising 5 or 6 members, such as a pyrrole, a furan, a thiophene, a thiazole, an isothiazole, an oxazole, an isoxazole, an imidazole, a pyrazole, a triazole, a pyridine, a pyrimidine, an indole, a benzofuran, a benzothiophene, a benzothiazole, a benzoxazole, a benzimidazole, an indazole, a benzotriazole, a quinoline, an isoquinoline, a cinnoline, a quinazoline, a quinoxaline, a carbazole, or a julolidine.

As well, the term "5-membered aromatic ring" refers to an aromatic hydrocarbon group ring such as furan, thiophene, oxazole, oxadiazole, isoxazole, thiazole, pyrrole, imidazole, pyrazole.

Also, the term "6-membered aromatic ring" refers to a phenyl, whereas "6-membered heteroaromatic ring" wherein the atoms of the ring(s) comprise one or more, advantageously 1 to 3 nitrogen atom . It can be for example benzene, pyridine, pyrazine, pyrimidine, pyridazine, triazine, or tetrazine.

The term "aryl-(C1-C6)-alkyl" as used herein refers to any aryl group as stated above, which is bound to the molecule by means of a (C1-C6)-alkyl group as defined above. In particular, it can be a phenyl group.

The term "heteroaryl-(C1-C6)alkyl" as used herein refers to a heteroaryl group as defined above, which is bound to the molecule by means of a (C1-C6)-alkyl group as defined above.

The term "oxo group", corresponds to the functional group '=O' wherein the substituent oxygen atom is connected to the carbon which bears it through a double bond.

The designation of a compound is intended to designate the compound itself, as well as any pharmaceutically acceptable salt, solvate, or stereoisomer thereof. In a preferred embodiment, the designation of a compound is intended to designate the compound as specifically designated in itself, as well as any pharmaceutically acceptable salt thereof.

The expression "salt and/or solvate" as used herein refer to a salt or solvate of a compound according to the invention, preferably pharmaceutically acceptable and having the pharmacological activity of the corresponding compound. Thus, within the meaning of the invention, the term "salt" refers to a pharmaceutically acceptable, inorganic or organic, acid or base addition salt of a compound of the present invention. The formation of a salt typically consists of associating an acidic, basic, or zwitterionic molecule with a counterion to create a salt version of the compound. A wide variety of chemical species can be used in the neutralization reaction. Although most salts of a given active ingredient are bioequivalent, some may have, among other things, increased solubility or bioavailability properties. Salt selection is now a common operation in the drug development method as taught by H. Stahl and C.G. Wermuth (Stahl *et a*/., 2011). Within the meaning of the invention, the term "solvates" corresponds to conventional solvates, such as those produced in the last stage of the preparation of the compounds of the invention due to the presence of solvents. For example, solvates due to the presence of water (these solvates are also called hydrates) or ethanol may be mentioned.

"Pharmaceutically acceptable" is meant to designate what is useful for the preparation of a pharmaceutical composition and is generally safe and non-toxic for pharmaceutical use.

An "effective dose", as used herein refers to an amount sufficient to induce a positive modification in the condition to be regulated or treated, but low enough to avoid serious side effects. An effective dose may vary with the particular condition being treated, the age and physical condition of the subject to be administered with the compounds of the invention, the severity of the infection to coronavirus, especially Sars-CoV2, to be treated or prevented; also an effective dose may vary as a function of the virulence of said coronavirus, in other words, the speed of multiplication and of invasion (spread) of the virus in the organism. The duration of the treatment, coadministration of other treatments, formulation which is employed, the route of administration, and like factors may also have an influence on the effective dose. Such factors are well known from the skilled in the art.

The expression "subject in need thereof" refers to any animal susceptible of suffering from a condition induced or caused by an infection to a coronavirus. More particularly, a subject in need thereof refers to any mammalian or avian subject. Even more particularly, said subject is a human subject. In particular, a subject in need thereof can be a subject who is or is at risk of being infected or exposed to a coronavirus, whether or not suffering from a condition or symptom induced or caused by a coronavirus. In other words, the subject can be symptomatic or asymptomatic. A subject infected or at risk of being infected by a coronavirus comprises a subject, a biological sample from which has been found to contain coronavirus particles and/or coronavirus peptide and/or coronavirus genetic material. A subject infected or at risk of being infected by a coronavirus comprises a subject who is or has been in touch with another subject infected by a coronavirus. Even more particularly said subject in need thereof is or is at risk of being infected by SARS-CoV-2.

The terms "prevention", "prophylaxis" or "preventing" as used herein when related to therapeutic applications, mean at least partly reducing the probability of occurrence of a condition or a symptom thereof, i.e., a condition or a symptom in relation to an infection to a coronavirus or even at least partially reducing the probability of occurrence of an infection (i.e. the multiplication of the virus in the organism) to a coronavirus.

The terms "treatment" or "treating" as used herein when related to therapeutic applications, mean at least partly curing a condition or a symptom thereof in relation to an infection to a coronavirus, i.e., a condition or a symptom in relation to an infection to a coronavirus, or even at least partly curing an infection to a coronavirus (in other words, at least diminishing or even suppressing the viral load of a coronavirus in the treated subject).

The term "coronavirus" as used herein refers to viruses of the family of the coronaviradae. Coronaviruses are generally specific to a host, either a mammal or an avian host but host specificity can change following mutations. More preferably the term coronavirus encompasses coronaviruses capable of infecting human beings. Even more preferably, it refers to SARS-CoV, MERS-CoV or to SARS-Cov-2 or any variants thereof. More specifically SARS-Cov-2, refers to any variants of SARS-CoV-2, for example Alpha, Beta, Gamma, Delta, Eta, Iota, Kappa, Lambda, Mu, Epsilon, Zeta or Theta variants according to labels from WHO.

Conditions and symptoms induced by the coronaviruses vary as a function of the tropism of the coronavirus. Symptoms are mostly either intestinal or respiratory, but also nervous. Coronaviruses can be responsible for colds, fever, sore throat, diarrhoea, pneumopathy, anosmia, ageusia and even severe pneumopathy leading to dyspnea, hypoxia, respiratory failure or even shock and/or multiorgan dysfunction. More specifically symptoms of COVID-19 are variable : infected subject can experience no noticeable symptom, but also fever, cough, headache, fatigue, breathing difficulties, mild pneumonia, or in the most severe cases dyspnea, hypoxia, respiratory failure or even shock and/or multiorgan dysfunction. More particularly, "a condition related to a coronavirus" refers to a pneumopathy as SARS (Severe Acute Respiratory Syndrome), MERS (Middle East respiratory syndrome), or to COVID-19 (Coronavirus disease 2019).

3C-like protease (3CLpro) refers to main protease (Mpro) of coronaviruses that is responsible for the generation of the majority the viral proteins from the polypeptide encoded by the genome of said coronaviruses. More particularly, 3CLpro refers to main protease of SARS-CoV, MERS-CoV or of SARS-Cov-2. Even more particularly, "3CLpro" refers to main protease of SARS-Cov-2. Of note, 3CLpro is produced as a part of a multifunctional polyprotein involved in the transcription and replication of viral RNAs and contains the proteases responsible for the cleavages of the polyprotein. 3CL-PRO is produced by the self-cleavage of the polyprotein which results in the production of peptide of (SEQ ID NO:1, from aa 3241 -3546 of Replicase polyprotein 1ab UniProtKB - P0C6U8 (R1A_SARS), (SEQ ID NO:2, from aa 3248 - 3553 of 2'-O-methyltransferase, UniProtKB - T2B9U0 (T2B9U0_MERS)) or (SEQ ID NO:3 from aa 3264-3569 of Replicase polyprotein 1ab, UniProtKB ID - P0DTD1 (R1AB_SARS2) for SARS-CoV, MERS-CoV or SARS-Cov-2 respectively.

### 3CLpro of SARS-CoV (SEQ ID NO:1) :

SGFRKMAFPSGKVEGCMVQVTCGTTTLNGLWLDDTVYCPRHVICTAEDMLNPNYEDLLIRKSN
HSFLVQAGNVQLRVIGHSMQNCLLRLKVDTSNPKTPKYKFVRIQPGQTFSVLACYNGSPSGVY
QCAMRPNHTIKGSFLNGSCGSVGFNIDYDCVSFCYMHHMELPTGVHAGTDLEGKFYGPFVDR
QTAQAAGTDTTITLNVLAWLYAAVINGDRWFLNRFTTTLNDFNLVAMKYNYEPLTQDHVDILGP
LSAQTGIAVLDMCAALKELLQNGMNGRTILGSTILEDEFTPFDVVRQCSGVTFQ.

### 3CLpro of MERS-CoV (SEQ ID NO:2) :

SGLVKMSHPSGDVEACMVQVTCGSMTLNGLWLDNTVWCPRHVMCPADQLSDPNYDALLISM
TNHSFSVQKHIGAPANLRVVGHAMQGTLLKLTVDVANPSTPAYTFTTVKPGAAFSVLACYNGR
PTGTFTVVMRPNYTIKGSFLCGSCGSVGYTKEGSVINFCYMHQMELANGTHTGSAFDGTMYG
AFMDKQVHQVQLTDKYCSVNVVAWLYAAILNGCAWFVKPNRTSVVSFNEWALANQFTEFVGT
QSVDMLAVKTGVAIEQLLYAIQQLYTGFQGKQILGSTMLEDEFTPEDVNMQIMGVVMQ.

### 3CLpro of SARS-Cov-2 (SEQ ID NO:3) :

"3CLpro of a coronavirus" as used herein is a protein whose sequence shares at least 80% homology with the 3CLpro protease of SEQ ID NO:1, of SEQ ID NO:2 or of SEQ ID NO:3. Preferably, the amino acid sequences of the homologs of the 3CLpro are identical at more than 80%, preferably 81%, more preferably 82%, more preferably 83%, more preferably 84%, more preferably 85%, preferably 86%, more preferably 87%, more preferably 88%, more preferably 89%, more preferably 90%, more preferably 91%, more preferably 92%, more preferably 93%, more preferably 94%, more preferably 95%, more preferably 96% and even more preferably 97% to SEQ ID NO:1, SEQ ID NO:2 or SEQ ID NO:3. In a particular embodiment "3CLpro of a coronavirus" refers to a protein of SEQ ID NO:1, SEQ ID NO:2 or SEQ ID NO:3. In a preferred embodiment "3CLpro of a coronavirus" refers to a protein of sequence SEQ ID NO:3.

As used herein "inhibiting 3CLpro", "inhibition of 3CLpro" is intended to refer to the suppression or the reduction of the activity of the 3CL protease when compared to a control. Accordingly, a "3CLpro inhibitor" is a compound which through interaction with 3CLpro is capable to suppress or at least to reduce 3CLpro peptidase activity, compared with said activity without the presence of said 3CLpro inhibitor. Said inhibitor can be either a covalent binder or a non-covalent binder and, accordingly, a reversible or irreversible inhibitor, respectively. A 3CLpro irreversible inhibitor binds and then bonds to 3CLpro, to permanently silencing or altering its activity. A 3CLpro reversible inhibitor binds and then bonds to 3CLpro, to permanently silencing or altering its activity. A reversible inhibitor of 3CLpro binds the enzyme with non-covalent interactions which can more or less strong depending on the compound and the number and / or kind of the interactions between the compound and 3CLpro.

### Compounds of the invention

Using proprietary platform of computational chemistry, all atom molecular dynamics modulation and based on their knowledge of structural insights of 3CLpro, inventors have been able to identify and refine a novel family of compounds which are particularly adapted to bind substrate-binding pocket of 3CLpro of SARS-CoV-2. These compounds are potent inhibitors of SARS-CoV-2 3CLpro and therefore constitute valuable candidates in the treatment and prophylaxis of COVID-19 and thought particularly adapted to inhibit SARS-CoV-2 3CLpro, they constitute valuable candidate treatments for other coronavirus infections.

According to a particular embodiment, compounds of the invention are of the following formula I as stated above, 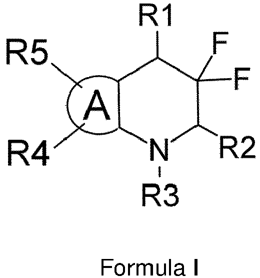 wherein A R1, R2, R3, R4, R5 are as stated above, in any enantiomeric, diastereomeric form, or any salt and/or solvate thereof.

In a more particular embodiment, in said compounds of formula I, R₁ and R₂ are each independently selected from a hydrogen atom, (C₁-C₆) alkyl, OH, OR₆, and an oxo group, preferably from a hydrogen atom, (C₁-C₆) alkyl, OH, and an oxo group, even more preferably from OH or an oxo group. In a particular embodiment R₁ and R₂ are each independently selected from OH or an oxo group. In a more particular embodiment R₁ and R₂ are different and selected from OH or an oxo group. In an even more particular embodiment, in said compounds, both R₁ and R₂ is an oxo group, said compounds constitute covalent inhibitors of 3CLpro peptidase activity which is of particular interest. In a further particular embodiment, in compounds of formula I, R1 is a OH, such compounds display a specific binding activity when compared to the other and display significant inhibitory activity.

In another particular embodiment, in said compounds of formula I, R₃ a methyl. In a preferred embodiment R3 is H.

In a more particular embodiment, in said compounds of formula I, R4 is selected from OR₇, SOR₇, SR₇, more preferably SR₇, a steric hindrance at this place has been found advantageous.

Of note no particular difference in the occupancy of 3CLpro substrate pocket is observed as a function of enantiomeric form of the carbon bearing R1. Accordingly, in a particular embodiment, the asymmetric carbon atom number 4 (which bears R1) is of (S) configuration when R1 is different from oxo group. In another particular embodiment, the asymmetric carbon atom number 4 (which bears R1) is of (R) configuration when R1 is different from oxo group. In a further particular embodiment, compound of the invention is in the form of a racemic mixture when R1 is different from oxo group.

According to a particular embodiment compounds of the invention are selected from a subgroup of compounds of formula I of the following formula Ia : 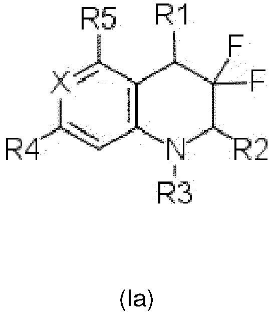 in any enantiomeric, diastereomeric form, or any salt and/or solvate thereof, wherein X is an atom selected from C or N, and wherein :
- R₁ and R₂ are each independently from the other selected from : a hydrogen atom, (C₁-C₆) alkyl, OH, OR₆, SR₆ and an oxo group,
- R₃ is selected from a hydrogen atom or a (C₁-C₆) alkyl,
- R₄ is selected from SO₂R₇, SONHR₇, OR₇, SOR₇, SR₇, (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
- R₅ is selected from H or a halogen atom,
- R₆ is a (C₁-C₆) alkyl, R₇ is selected from (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
with the proviso that at least one of R₁ or R₂ is different from an hydrogen atom.

In a more particular embodiment, in said compounds of formula la, R₁ and R₂ are each independently selected from a hydrogen atom, (C₁-C₆) alkyl, OH, OR₆, and an oxo group, preferably from a hydrogen atom, (C₁-C₆) alkyl, OH, and an oxo group, even more preferably from OH or an oxo group. In a particular embodiment R₁ and R₂ are each independently selected from OH or an oxo group. In a more particular embodiment R₁ and R₂ are different and selected from OH or an oxo group. In an even more particular embodiment, in said compounds, both R₁ and R₂ is an oxo group, said compounds constitute covalent inhibitors of 3CLpro peptidase activity. In a further particular embodiment, in compounds of formula la, R1 is a OH, such compounds display a specific binding activity when compared to the other and display significant inhibitory activity toward 3CLpro.

In another particular embodiment in said compounds of formula la, R₃ a methyl. In a preferred embodiment R₃ is H.

In another particular embodiment, in said compounds of formula la, R₄ is selected from OR₇, SOR₇, SR₇, more preferably SR₇, a steric hindrance at this place has been found advantageous.

In another particular embodiment, R5 is selected from H or a fluorine atom.

In a particular embodiment, the asymmetric carbon atom number 4 (which bears R1), in said compounds of formula la, is of (S) configuration when R₁ is different from oxo group.

In another particular embodiment, the asymmetric carbon atom number 4 (which bears R1) in said compounds of formula la, is of (R) configuration when R₁ is different from oxo group.

In a further particular embodiment, said compounds of formula Ia according to the invention is in the form of a racemic mixture when R₁ is different from oxo group.

Among the compounds of general formula (Ia) according to the invention, the following compounds 1 to 18 constitute particular specific embodiments:

**Table I**

| **Compound number** | **STRUCTURE** | **CHEMICAL NAME** |
|---|---|---|
| **1** | 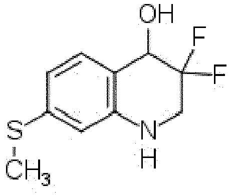 | 3,3-difluoro-7-(methylsulfanyl)-1,2,3,4-tetrahydroquinolin-(R)-4 -ol |
| **2** | 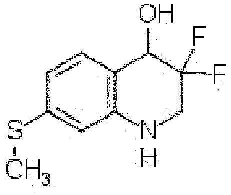 | 3,3-difluoro-7-(methylsulfanyl)-1,2,3,4-tetrahydroquinolin-(S)-4 -ol |
| **3** | 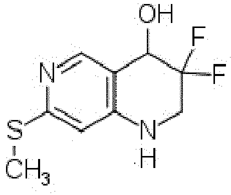 | 3,3-difluoro-7-(methylsulfanyl)-1,2,3,4-tetrahydro-1,6-naphthyridin-4-(R)-ol |
| **4** | 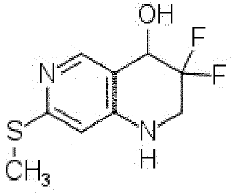 | 3,3-difluoro-7-(methylsulfanyl)-1,2,3,4-tetrahydro-1,6-naphthyridin-4-(S)-ol |
| **5** | 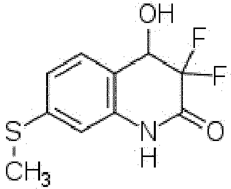 | 3,3-difluoro-(R)-4-hydroxy-7-(methylsulfanyl)-1,2,3,4-tetrahydroquinolin-2-one |
| **6** | 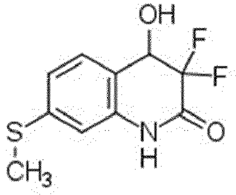 | 3,3-difluoro-(R)-4-hydroxy-7-(methylsulfanyl)-1,2,3,4-tetrahydroquinolin-2-one |
| **7** | 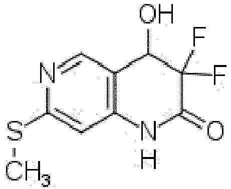 | 3,3-difluoro-(R)-4-hydroxy-7-(methylsulfanyl)-1,2,3,4-tetrahydro-1, 6-naphthyridin-2-one |
| **8** | 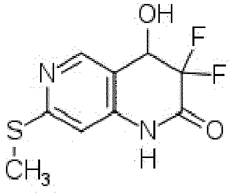 | 3,3-difluoro-(S)-4-hydroxy-7-(methylsulfanyl)-1,2,3,4-tetrahydro-1, 6-naphthyridin-2-one |
| **9** | 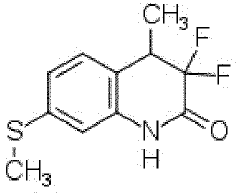 | 3,3-difluoro-(S)-4-methyl-7-(methylsulfanyl)-1,2,3,4-tetrahydroquinolin-2-one |
| **10** | 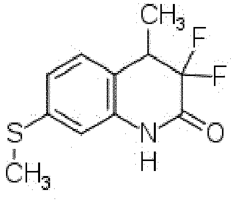 | 3,3-difluoro-(R)-4-methyl-7-(methylsulfanyl)-1,2,3,4-tetrahydroquinolin-2-one |
| **11** | 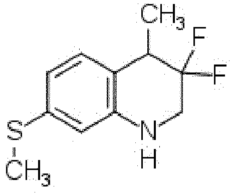 | 3,3-difluoro-(S)-4-methyl-7-(methylsulfanyl)-1,2,3,4-tetrahydroquinoline |
| **12** | 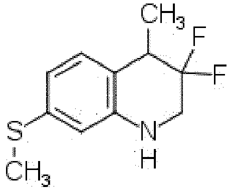 | 3,3-difluoro-(R)-4-methyl-7-(methylsulfanyl)-1,2,3,4-tetrahydroquinoline |
| **13** | 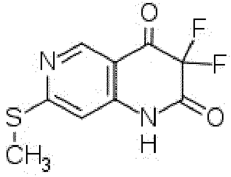 | 3,3-difluoro-7-(methylsulfanyl)-1,2,3,4-tetrahydro-1,6-naphthyridine-2,4-dione |
| **14** | 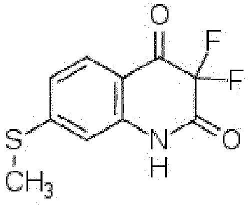 | 3,3-difluoro-7-(methylsulfanyl)-1,2,3,4-tetrahydroquinoline-2,4-dione |
| **15** | 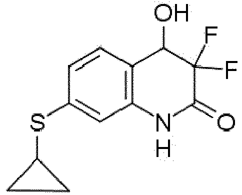 | 3,3-difluoro-(R)-4-hydroxy-7-(cyclopropylsulfanyl)-1,2,3,4-tetrahydroquinoline-2-one |
| **16** | 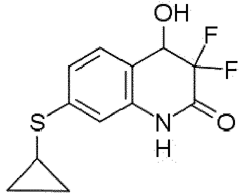 | 3,3-difluoro-(S)-4-hydroxy-7-(cyclopropylsulfanyl)-1,2,3,4-tetrahydroquinoline-2-one |
| **17** | 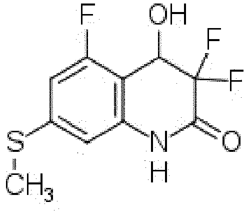 | 3,3,5-trifluoro-(R)-4-hydroxy-7-(methylylsulfanyl)-1,2,3,4-tetrahydroquinoline-2-one |
| **18** | 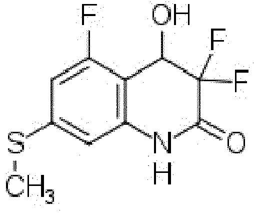 | 3,3,5-trifluoro-(S)-4-hydroxy-7-(methylylsulfanyl)-1,2,3,4-tetrahydroquinoline-2-one |

Accordingly, in a particular embodiment, a compound according to the invention is selected from compounds 1 to 18 indicated here-above.

According to a particular embodiment compounds of the invention are selected from compounds of the following formula Ib : 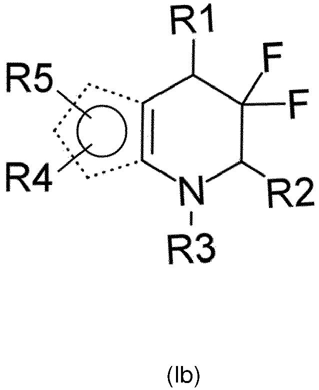 in any enantiomeric, diastereomeric form, or any salt and/or solvate thereof, wherein :
- R₁ and R₂ are each independently from the other selected from : a hydrogen atom, (C₁-C₆) alkyl, OH, OR₆, SR₆ and an oxo group,
- R₃ is selected from a hydrogen atom or a (C₁-C₆) alkyl,
- R₄ is selected from SO₂R₇, SONHR₇, OR₇, SOR₇, SR₇, (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
- R₅ is selected from H or a halogen atom,
- R₆ is a (C₁-C₆) alkyl, R₇ is selected from (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
with the proviso that at least one of R₁ or R₂ is different from an hydrogen atom.

In a particular embodiment, in these compounds of formula Ib, the 5 membered aromatic cycle is selected from furan, thiophene, oxazole, oxadiazole, isoxazole, thiazole, pyrrole, imidazole, pyrazole. More preferably, the 5 membered aromatic cycle is a pyrazole.

In a more particular embodiment, in said compounds of formula la, R₁ and R₂ are each independently selected from a hydrogen atom, (C₁-C₆) alkyl, OH, OR₆, and an oxo group, preferably from a hydrogen atom, (C₁-C₆) alkyl, OH, and an oxo group, even more preferably from OH or an oxo group. In a particular embodiment R₁ and R₂ are each independently selected from OH or an oxo group. In a more particular embodiment R₁ and R₂ are different and selected from OH or an oxo group. In an even more particular embodiment, in said compounds, both R₁ and R₂ is an oxo group. In a further particular embodiment, in compounds of formula Ib, R1 is a OH, such compounds display a specific binding activity when compared to the other and display significant inhibitory activity.

In another particular embodiment in said compounds of formula la, R₃ a methyl In a preferred embodiment R3 is H.

In a more particular embodiment, in said compounds of formula Ib, R4 is selected from OR₇, SOR₇, SR₇, more preferably SR₇.

In a particular embodiment, the asymmetric carbon atom number 4 (which bears R1), in said compounds of formula Ib, is of (S) configuration when R1 is different from oxo group.

In another particular embodiment, the asymmetric carbon atom number 4 (which bears R1) in said compounds of formula Ib, is of (R) configuration when R1 is different from oxo group.

In a further particular embodiment, said compounds of formula Ib according to the invention is in the form of a racemic mixture when R1 is different from oxo group

Among the compounds of general formula (Ib) according to the invention, the following compounds 19 to 25 constitute particular specific embodiments:

**Table II**

| **Compound number** | **STRUCTURE** | **NAME** |
|---|---|---|
| **19** | 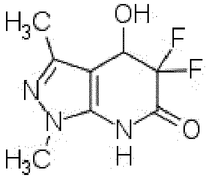 | 5,5-difluoro-(R)-4-hydroxy-1,3-dimethyl-1H,4H,5H,6H,7H-pyrazolo [3,4-b]pyridin-6-one |
| **20** | 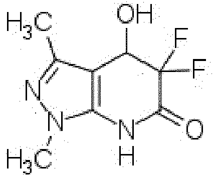 | 5,5-difluoro-(S)-4-hydroxy-1,3-dimethyl-1H,4H,5H,6H,7H-pyrazolo [3,4-b]pyridin-6-one |
| **21** | 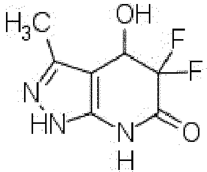 | 5,5-difluoro-(R)-4-hydroxy-3-methyl-1H,4H,5H,6H,7H-pyrazolo[3,4 -b]pyridin-6-one |
| **22** | 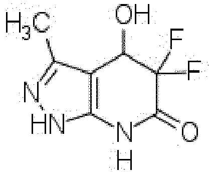 | 5,5-difluoro-(S)-4-hydroxy-3-methyl-1H,4H,5H,6H,7H-pyrazolo[3,4 -b]pyridin-6-one |
| **23** | 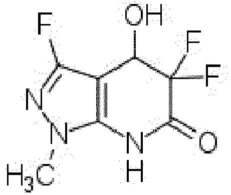 | 3,5,5-trifluoro-(R)-4-hydroxy-1-methyl-1H,4H,5H,6H,7H-pyrazolo[3,4 -b]pyridin-6-one |
| **24** | 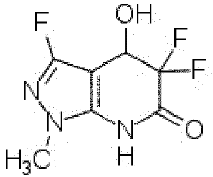 | 3,5,5-trifluoro-(S)-4-hydroxy-1-methyl-1H,4H,5H,6H,7H-pyrazolo[3,4 -b]pyridin-6-one |
| **25** | 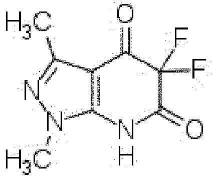 | 5,5-difluoro-1,3-dimethyl-1H,4H,5H,6H,7H-pyrazol o[3,4-b]pyridine-4,6-dione |

Accordingly, in a particular embodiment, a compound according to the invention is selected from compounds 19 to 25 indicated here-above.

Inhibitory activity actvity of the compounds of the invention can be tested *in vitro* using any of the methods well known in the art, and notably those described in the experimental section. Covalent or no covalent binding can be easily determined by methods well known in the art, for example as such as ESI-MS which is described in experimental section.

### Therapeutic applications of compounds of the invention

As mentioned above and illustrated by the following examples, the compounds according to the present invention are useful as inhibitors of 3CLpro proteinase of coronaviruses, and more specifically of 3CLpro of SARS-CoV-2.

Indeed, inventors have been able to identify a novel family of 3CLpro inhibitors which, as shown in the example section, show an efficient binding to 3CLpro and an efficient inhibitory effect for 3CLpro proteolytic activity. As a consequence, they are of a particular interest in the therapeutic field. Though all the compounds described herein are of interest, covalent inhibitors as described above are of particular interest.

Accordingly, in an aspect, the present invention therefore relates to a compound of the invention as described above, for its use as a medicament, in particular a medicament intended to inhibit the activity of 3CLpro.

In an embodiment, the invention relates to the use of a compound according to the invention for the preparation of a medicine, in particular of a drug for inhibiting the 3CLpro of coronaviruses.

In a further embodiment, the present invention relates to a medicament comprising at least one compound according to the invention, in particular a medicament for inhibiting the activity of 3CLpro of coronaviruses.

3C-like protease (3CLpro) is the main protease that is pivotal for the replication of the coronaviruses, and especially SARS-Cov-2 in the host cells. Also, compounds of the invention which are efficient inhibitors of this protease provide valuable candidates in lowering or even stopping the infection cycle, especially in regard with SARS-CoV-2 infections, because they have been designed and selected based on their ability to fully fill the substrate binding pocket of SARS-CoV-2. Such treatments are of particular use for subjects showing no immunological response to vaccines, with an impaired immune system resulting in a less effective vaccine protection because of a shorter period of production or a lower production of neutralizing (protective) antibodies and/or for subjects for whom vaccination is contraindicated. Also, these treatments are of particular use in subjects having a higher risk, when infected (even if vaccinated), of experiencing a severe form of the disease, as severe pneumopathy or respiratory failure. More particularly, these subjects comprise : 65-years-old subjects or older, pregnant females, subjects with a body mass index ≥ 30, subjects with congenital or acquired immunosuppression and/or subjects suffering from : progressive cancer under treatment cardiovascular diseases, from complicated diabetes, from chronic respiratory conditions at risk to decompensate during a viral infection (e.g. obstructive pulmonary disease, severe asthma, pulmonary fibrosis, sleep apnea syndrome, cystic fibrosis), from chronic renal failure on dialysis, from cirrhosis (of at least stage B of Child Pugh score), from a major sickle cell syndrome or with a history of splenectomy, from neuromuscular diseases that affect the respiratory functions.

Compounds of the invention are also of particular interest in case of the advent of a variant of coronavirus, especially variant of SARS-CoV2, that would escape at least in part to the immune response induced by the existing vaccines.

Compounds of the invention which are small molecules, provide an affordable alternative to monoclonal antibodies therapies which are quite expensive (around 2000 euros a dose) because of production costs.

Accordingly, in an embodiment, the invention relates to a compound of the invention as defined above, for its use in the prevention, the treatment, or combination thereof, of an infection to a coronavirus. In a more particular embodiment, said coronavirus is selected from SARS-CoV, MERS-CoV or SARS-Cov-2, more preferably said coronavirus is SARS-Cov-2.

According to another embodiment, the invention relates to a compound of the invention as defined above, for its use in the prophylaxis, the treatment, or combination thereof, of a condition or symptom resulting from an infection to a coronavirus. In a more particular embodiment, said coronavirus is selected from SARS-CoV, MERS-CoV or SARS-Cov-2, more preferably said coronavirus is SARS-Cov-2. In particular, said condition and/or symptom is selected from : cold, fever, sore throat, diarrhoea, pneumopathy, anosmia, ageusia, severe pneumopathy, dyspnea, hypoxia, respiratory failure, even shock, multiorgan dysfunction, SARS, MERS, COVID-19.

In an embodiment, the invention relates to a compound selected from any one of the compounds listed in table I or II as defined above for its use as a medicament.

In another embodiment, the invention relates to a compound selected from any one of the compounds listed in table I or II as defined above for its use in the prophylaxis, the treatment, or combination thereof, of an infection to a coronavirus.

In an even further embodiment, the invention relates to a compound selected from any one of the compounds listed in table I or II as defined above for its use in the prophylaxis, the treatment, or combination thereof, of a condition or symptom resulting from a coronavirus. More particularly said condition or symptom is selected from cold, fever, sore throat, diarrhoea, pneumopathy, anosmia, ageusia, severe pneumopathy, dyspnea, hypoxia, respiratory failure, even shock, multiorgan dysfunction, SARS, MERS, COVID-19.

In a further embodiment of the invention, the invention relates to a compound selected from any one of the compounds listed in table I or II as defined above for its use in the prophylaxis, the treatment, or combination thereof, of COVID-19.

According to another aspect, the invention is related to a method for preventing and/or treating of a condition or symptom resulting from a coronavirus, said method comprising at least a step of administering to a subject in need thereof at least an effective amount of at least one compound according to the invention, as specified above. More particularly said condition or symptom is selected from cold, fever, sore throat, diarrhoea, pneumopathy, anosmia, ageusia, severe pneumopathy, dyspnea, hypoxia, respiratory failure, even shock, multiorgan dysfunction, SARS, MERS, COVID-19.

In an embodiment, the invention is related to a method for preventing and/or treating an infection to a coronavirus, said method comprising at least a step of administering to a subject in need thereof at least an effective amount of at least one compound according to the invention.

In a more particular embodiment, the invention relates to a method for preventing and/or treating COVID-19, said method comprising at least a step of administering to a subject in need thereof at least an effective amount of at least one compound according to the invention.

In an even more particular embodiment, the above methods of preventing and treating comprise at least a step of administering to a subject in need thereof at least an effective amount of at least one compound selected from any one of the compounds listed in table I or II.

In a further embodiment, the above methods of preventing and treating comprise, prior to the step of administering, a step of detecting, in a biological sample of a subject, at least a coronavirus particle and/or a coronavirus peptide and/or a coronavirus genetic material.

According to a further aspect, the present invention relates to a pharmaceutical composition comprising at least one compound according to the invention as defined above, and at least one pharmaceutically acceptable excipient.

In a particular embodiment, said pharmaceutical composition contains an effective dose of at least one compound according to the invention defined above.

According to one embodiment, a pharmaceutical composition of the invention may be intended to be administered separately, sequentially or simultaneously with an agent useful for the prevention and/or the treatment of condition or symptom caused by a coronavirus, in particular SARS-CoV2, said agent being different from the compound of formula (I) of the invention.

In any of the therapeutics applications as described above, compounds 13 or 14, which, as shown in the experimental section, constitute particularly effective covalent inhibitors of 3CLpro of SARS-CoV-2, are particularly preferred.

### Compounds of the invention as biotechnological tools

Compounds of the invention provide valuable biotechnological tools for, for example, studying 3CLpro coronavirus enzymes, their substrate specificity, in screening for drugs candidates but also studying coronaviruses infection and infection cycle.

Also in an embodiment, the invention relates to the use of the compounds of the invention to *in vitro* inhibiting a viral 3-chymotrypsin-like protease (3CLpro), for example a 3CLpro of a coronavirus, for example of MERS-CoV, of a SARS-CoV, for example, a 3CLpro of SARS-CoV1 or a 3CLpro of SARS-CoV-2. In a particular embodiment, said compound is a covalent binder of 3CLpro. In another particular embodiment, said compound is a non-covalent binder of 3CLpro. Covalent or no covalent binding can be easily determined by methods well known in the art, for example as such as ESI-MS which is described in the experimental section.

### Preparation of the compounds of the invention

The compounds of the invention may be prepared by organic synthesis according to pathways well known from the skilled in the art.

In particular, example 1 of the experimental section illustrates the protocol of preparation of several compounds which can be easily derivable by the skilled in the art to obtain compounds according to the invention.

### EXAMPLES

### EXAMPLE 1: Preparation of the compounds according to the invention

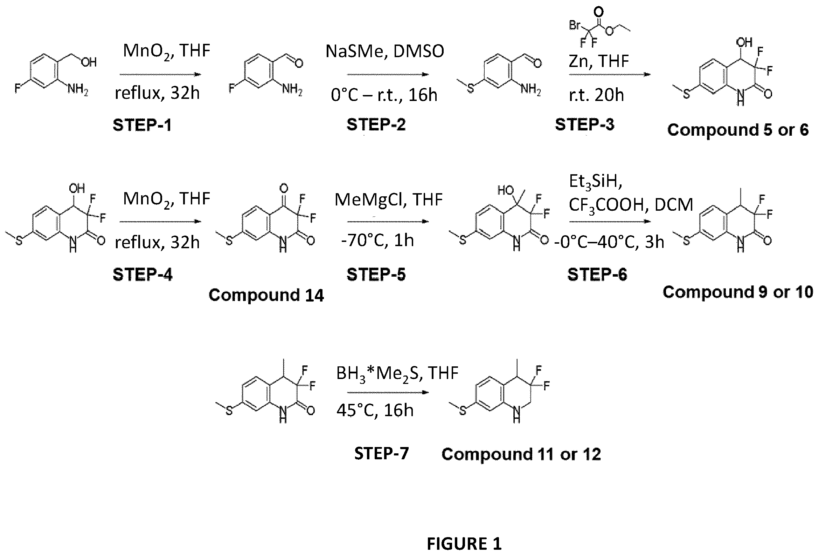

### Synthesis of compounds 5, 6, 9-12,14 (Figure 1)

### Step-1. Synthesis of 2-amino-4-fluorobenzaldehyde

A mixture of (2-amino-4-fluorophenyl)methanol (14.2 g, 100.61 mmol) and manganese(IV) oxide (52.48 g, 603.64 mmol) in THF (500 mL) was stirred several days (TLC control) at reflux. The solids were filtrated off and the filtrate was concentrated under reduced pressure to afford 2-amino-4-fluorobenzaldehyde (13.5 g, 97.03 mmol, 96.4% yield).

### Step-2. Synthesis of 2-amino-4-(methylsulfanyl)benzaldehyde

To an ice-cooled solution of 2-amino-4-fluorobenzaldehyde (16.0 g, 115.0 mmol) in DMSO (50 mL) sodium methylsulfanide (16.12 g, 230.01 mmol, 76.77 ml, 2.0 equiv) was added dropwise. Upon completion of the reaction, the mixture was allowed to warm up to ambient temperature and stirred overnight. The solution was diluted with water and extracted with EtOAc(3x50mL). The organic layer was washed with water, brine, dried over sodium sulfate, and evaporated under reduced pressure to give 2-amino-4-(methylsulfanyl)benzaldehyde (13.0 g, 77.74 mmol, 67.6% yield).

### Step-3. Synthesis of 3,3-difluoro-4-hydroxy-7-(methylsulfanyl)-1,2,3,4-tetrahydroquinolin-2-one (compound 5 or 6)

To the mixture of activated zinc (3.6 g, 55.01 mmol) in dry THF, ethyl 2-bromo-2,2-difluoroacetate (9.71 g, 47.84 mmol) was added and the mixture was stirred for 1 hour at room temperature. Then, 2-amino-4-(methylsulfanyl)benzaldehyde (4.0 g, 23.92 mmol) in THF was added dropwise and the reaction mixture was stirred for a further 19 h at room temperature. The mixture was diluted with ethyl acetate (25x2 mL) and was washed with water (50x2 mL) and brine (50x2 mL), was dried over sodium sulfate, and concentrated under reduced pressure to give pure 3,3-difluoro-4-hydroxy-7-(methylsulfanyl)-1,2,3,4-tetrahydroquinolin-2-one. Yield: 1000 mg, 17%; Appearance: Orange solid; ¹H NMR (500 MHz, DMSO-de) δ 10.93 (s, 1H), 7.34 (d, *J* = 8.1 Hz, 1H), 7.02 - 6.89 (m, 1H), 6.82 (s, 1H), 6.47 (d, *J* = 5.7 Hz, 1H), 5.00 - 4.84 (m, 1H), 2.44 (s, 3H); HPLC purity: 100%; LCMS: 246.0[M+H]⁺.

### Step-4. Synthesis of 3,3-difluoro-7-(methylsulfanyl)-1,2,3,4-tetrahydroquinoline-2,4-dione., (compound 14)

A mixture of 3,3-difluoro-4-hydroxy-7-(methylsulfanyl)-1,2,3,4-tetrahydroquinolin-2-one (1.3 g, 5.3 mmol), manganese(IV) oxide (4.61 g, 53.0 mmol) and THF (50 mL) was stirred several days (TLC control) at reflux. The solids were filtrated out and the filtrate was concentrated to afford the 3,3-difluoro-7-(methylsulfanyl)-1,2,3,4-tetrahydroquinoline-2,4-dione. Yield: 1000 mg, 77.6%; Appearance: Yellow solid; ¹H NMR (400 MHz, DMSO-de) δ 11.32 (s, 1H), 7.74 (d, *J* = 8.3 Hz, 1H), 7.06 (dd, *J* = 8.4, 1.8 Hz, 1H), 6.93 (d, *J* = 1.8 Hz, 1H), 2.54 (s, 3H); HPLC purity: 100%; LCMS: 244.0[M+H]⁺.

### Step-5. Synthesis of 3,3-difluoro-4-hydroxy-4-methyl-7-(methylsulfanyl)-1,2,3,4-tetrahydroquinolin-2-one.

Chloro(methyl)magnesium (322.8 mg, 4.32 mmol, 1.47 ml, 3.0 equiv) was added dropwise to a solution of 3,3-difluoro-7-(methylsulfanyl)-1,2,3,4-tetrahydroquinoline-2,4-dione (350.0 mg, 1.44 mmol) in 30 mL of THF at -70 °C under argon and reaction mixture was stirred at this temperature 1 hour. Then it was quenched with 30mL of saturated aqueous NH₄Cl solution and concentrated under vacuum. The residue was partitioned between 50 mL of water and 100 mL of EtOAc. The organic layer was washed with 20 mL of water, brine, dried over sodium sulfate, and concentrated under vacuum to give 3,3-difluoro-4-hydroxy-4-methyl-7-(methylsulfanyl)-1,2,3,4-tetrahydroquinolin-2-one. Yield: 350 mg, 86.3%; Appearance: Yellow solid; ¹H NMR (500 MHz, Chloroform-d) δ 8.58 (s, 1H), 7.47 (d, *J* = 8.2 Hz, 1H), 7.01 (dd, J *=* 8.1, 1.8 Hz, 1H), 6.75 (d, *J* = 1.9 Hz, 1H), 2.47 (s, 3H), 1.60 (s, 3H); HPLC purity: 100%; LCMS: 260.2[M+H]⁺.

### Step-6. Synthesis of 3,3-difluoro-4-methyl-7-(methylsulfanyl)-1,2,3,4-tetrahydroquinolin-2-one (compound 9 or 10).

3,3-Difluoro-4-hydroxy-4-methyl-7-(methylsulfanyl)-1,2,3,4-tetrahydroquinolin-2-one (308.01 mg, 1.19 mmol) was dissolved in DCM, the mixture was cooled to 0 °C and 2,2,2-trifluoroacetic acid (677.28 mg, 5.94 mmol, 460.0 µl, 5.0 equiv) and triethylsilane (691.6 mg, 5.95 mmol, 950.0 µl, 5.0 equiv) was added. The mixture was heated to 40 °C and stirred 3h at that temperature.

After that mixture was cooled to ambient temperature and was washed with water and aq. NaHCO₃ solution to give pure 3,3-difluoro-4-methyl-7-(methylsulfanyl)-1,2,3,4-tetrahydroquinolin-2-one. Yield: 200 mg, 69.2%; Appearance: Beige solid; ¹H NMR (600 MHz, DMSO-*d*₆) δ 10.98 (s, 1H), 7.24 (d, *J* = 8.0 Hz, 1H), 6.96 (dd, *J* = 8.0, 1.9 Hz, 1H), 6.84 (d, *J* = 1.9 Hz, 1H), 3.71 ― 3.50 (m, 1H), 2.43 (s, 3H), 1.24 (d, *J* = 7.1 Hz, 3H); HPLC purity: 100%; LCMS: 244.0[M+H]⁺.

### Step-7. Synthesis of 3,3-difluoro-4-methyl-7-(methylsulfanyl)-1,2,3,4-tetrahydroquinoline (compound 11 or 12).

To a solution of 3,3-difluoro-4-methyl-7-(methylsulfanyl)-1,2,3,4-tetrahydroquinolin-2-one (100.0 mg, 411.06 µmol) in 30 mL of dry THF borane dimethyl sulfide complex (93.73 mg, 1.23 mmol) was added in one portion. The resulting mixture was stirred at 45 °C overnight, then poured into cold K₂CO₃ aq. solution and extracted with EtOAc. The organic layer was washed with water, brine, dried over sodium sulfate, and evaporated under reduced pressure. The reside was subjected to HPLC to give 3,3-difluoro-4-methyl-7-(methylsulfanyl)-1,2,3,4-tetrahydroquinoline. Yield: 8.5 mg, 8.6%; Appearance: Yellow oil; ¹H NMR (400 MHz, DMSO-*d*₆) δ 7.15 (d, *J* = 8.0 Hz, 1H), 6.75 (dd, *J* = 8.1, 2.0 Hz, 1H), 6.70 (d, *J* = 2.0 Hz, 1H), 3.76 ― 3.62 (m, 1H), 3.62 - 3.53 (m, 1H), 3.39 - 3.23 (m, 1H), 2.55 (s, 3H), 1.51 (d, *J* = 7.0 Hz, 3H); HPLC purity: 100%; LCMS: 230.2[M+H]⁺.

### Synthesis of 3,3-difluoro-7-(methylsulfanyl)-1,2,3,4-tetrahydroquinolin-4-ol (compound 1 or 2):

To a solution of 3,3-difluoro-4-hydroxy-7-(methylsulfanyl)-1,2,3,4-tetrahydroquinolin-2-one (100.0 mg, 407.75 µmol) in 30 mL of dry THF borane dimethyl sulfide complex (92.93 mg, 1.22 mmol) was added in one portion. The resulting mixture was stirred at 45 °C overnight, then poured into cooled K₂CO₃ aqua solution and extracted with EtOAc. The organic layer was washed with water, brine, dried over sodium sulfate, and evaporated under reduced pressure. The resulting residue was subjected to HPLC purification to give 3,3-difluoro-7-(methylsulfanyl)-1,2,3,4-tetrahydroquinolin-4-ol. Yield: 48.9 mg, 51.9%; Appearance: Beige solid; ¹H NMR (400 MHz, Methanol-*d*₄) δ 7.12 (d, *J* = 8.0 Hz, 1H), 6.57 (dd, *J* = 8.0, 1.8 Hz, 1H), 6.52 (d, *J* = 1.8 Hz, 1H), 4.56 (t, *J* = 7.6, 6.7 Hz, 1H), 3.69 - 3.51 (m, 1H), 3.42 - 3.34 (m, 1H), 2.42 (s, 3H); HPLC purity: 100%; LCMS: 232.0[M+H]⁺.

### Synthesis of 5,5-difluoro-4-hydroxy-1,3-dimethyl-1H,4H,5H,6H,7H-pyrazolo[3,4-b]pyridin-6-one (compound 19 or 20):

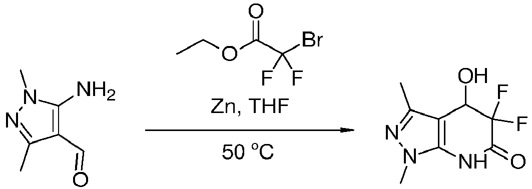

To an oven-dried 250 ml tube was charged with zinc (587.49 mg, 8.98 mmol) in dry THF. To activate the Zn, the initiator 1,2-dibromoethane (6.69 mg, 35.59 µmol) was added and the reaction mixture was heated to reflux and then was cooled to 50 °C. THF solution of 5-amino-1,3-dimethyl-1H-pyrazole-4-carbaldehyde (500.0 mg, 3.59 mmol) and ethyl 2-bromo-2,2-difluoroacetate (1.46 g, 7.19 mmol) was added dropwise at 50 °C. After completion of the reaction, the reaction was cooled to room temperature and filtered through Celite. Then the organic layer was separated, and the water phase was extracted with EtOAc 3 times. The combined organic phase was evaporated under reduced pressure to provide the compound 5,5-difluoro-4-hydroxy-1,3-dimethyl-1H,4H,5H,6H,7H-pyrazolo[3,4-b]pyridin-6-one. Yield: 29.1 mg, 3.8%; Appearance: Colorless solid; ¹H NMR (400 MHz, DMSO-de) δ 11.61 (s, 1H), 6.02 (d, *J* = 5.6 Hz, 1H), 4.83 - 4.64 (m, 1H), 3.61 (s, 3H), 2.09 (s, 3H), HPLC purity: 94.53%; LCMS: 218.0[M+H]⁺.

### Synthesis of compounds 7, 8 and 13:

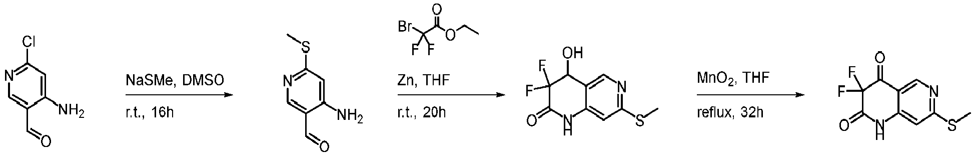

### Step-1. Synthesis of 4-amino-6-(methylsulfanyl)pyridine-3-carbaldehyde

To an ice cooled solution of 4-amino-6-chloropyridine-3-carbaldehyde (20.0 g, 127.74 mmol) in DMSO (50 mL) sodium methylsulfanide (26.86 g, 383.22 mmol, 127.9 mL, 3.0 equiv) was added dropwise. Upon completion the mixture was allowed to warm up to rt and stir overnight. The solution was diluted with water and extracted with EtOAc (3x50mL). The organic layer was washed with water, brine, dried and evaporated in vacuo to give 4-amino-6-(methylsulfanyl)pyridine-3-carbaldehyde (13.0 g, 77.28 mmol, 60.5% yield).

### Step-2. Synthesis of 3,3-difluoro-4-hydroxy-7-(methylsulfanyl)-1,2,3,4-tetrahydro-1,6-naphthyridin-2-one (compound 7 or 8).

To the mixture of activated zinc (6.84 g, 104.63 mmol) in THF at room temperature. Ethyl 2-bromo-2,2-difluoroacetate (19.91 g, 98.09 mmol) was added and stirred for 1h at room temperature. To this mixture 4-amino-6-(methylsulfanyl)pyridine-3-carbaldehyde (5.5 g, 32.7 mmol) in THF was added dropwise and the reaction mixture was stirred for a further 19h at room temperature. Dilute the mixture with 25 x2 mL of ethyl acetate and wash with water (50x2 mL) and brine (50x2 mL), dry over sodium sulphate and concentrate in vacuo to give pure 3,3-difluoro-4-hydroxy-7-(methylsulfanyl)-1,2,3,4-tetrahydro-1,6-naphthyridin-2-one. Yield: 2190 mg, 27.2%; Appearance: White solid; ¹H NMR (400 MHz, DMSO-de) δ 11.32 (s, 1H), 8.36 (s, 1H), 6.77 (s, 1H), 6.64 (d, *J* = 5.4 Hz, 1H), 5.14 - 5.03 (m, 1H), 2.50 (s, 3H); HPLC purity: 97.3%; LCMS Calculated for C₉H₈F₂N₂O₂S: 247.03; Observed: 247.0[M+H]⁺.

### Step-3. Synthesis of 3,3-difluoro-7-(methylsulfanyl)-1,2,3,4-tetrahydro-1,6-naphthyridine-2,4-dione (compound 13)

A mixture of 3,3-difluoro-4-hydroxy-7-(methylsulfanyl)-1,2,3,4-tetrahydro-1,6-naphthyridin-2-one (300.0 mg, 1.22 mmol), manganese (IV) oxide (636.49 mg, 7.32 mmol) and THF (50 mL) was stirred several days (TLS control) at reflux. The solids were filtrated out and the filtrate was concentrated to afford the 3,3-difluoro-7-(methylsulfanyl)-1,2,3,4-tetrahydro-1,6-naphthyridine-2,4-dione, QUB-00005_Int_07. Yield: 79.3 mg, 26.1%; Appearance: Yellow solid; ¹H NMR (500 MHz, DMSO-de) δ 11.68 (s, 1H), 8.74 (s, 1H), 6.88 (s, 1H), 2.56 (s, 3H); HPLC purity: 100%; LCMS Calculated for C₉H₆F₂N₂O₂S: 244.01; Observed: 243.0[M-H]⁻.

### Synthesis of 3,3-difluoro-7-(methylsulfanyl)-1,2,3,4-tetrahydro-1,6-naphthyridin-4-ol, (compound 3 or 4):

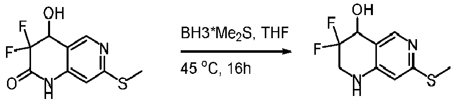

To a solution of 3,3-difluoro-4-hydroxy-7-(methylsulfanyl)-1,2,3,4-tetrahydro-1,6-naphthyridin-2-one (compound 7 or 8199.32 mg, 809.47 µmol) in 30 mL of dry THF borane dimethyl sulfide complex (184.0 mg, 2.42 mmol, 230.0 µl, 3.0 equiv) was added in one portion. The resulting mixture was stirred at 45 C overnight, then poured into cold K₂CO₃ water solution and extracted with EtOAc. The organic layer was washed with water, brine, dried and evaporated in vacuo to give 3,3-difluoro-7-(methylsulfanyl)-1,2,3,4-tetrahydro-1,6-naphthyridin-4-ol, QUB-00005_Int_01. Yield: 3.3 mg, 1.7%; Appearance: Beige solid; ¹H NMR (400 MHz, Methanol-*d*₄) δ 7.98 (s, 1H), 6.43 (s, 1H), 4.67 - 4.59 (m, 1H), 3.74 - 3.59 (m, 1H), 3.52 - 3.40 (m, 1H), 2.46 (s, 3H); HPLC purity: 100%; LCMS Calculated for C₉H₈F₂N₂O₂S: 233.06; Observed: 247.2[M+H]⁺.

### EXAMPLE 2: Assessment of the binding and inhibitory activities of the compounds of the invention

### METHODS

### Production and purification of recombinant SARS-Cov-2

The plasmid PGEX-6p-1 encoding SARS-CoV-2 3CLpro was used (Zhang, L. et al., 2020). Protein expression and purification protocol retraced the procedure reported by Zhang, L. et al. (2020) (where structure of 3CLpro 6Y2E in the canonical form was presented). The plasmid was transformed into *E. coli* strain BL21 (DE3) and then pre-cultured in YT medium at 37°C overnight in presence of 100 µg/mL ampicillin. The following expression culture was grown at 37 °C and induced at OD600 0.6-0.8 with before induction of overexpression with 0.5 mM isopropyl-D-thiogalactoside (IPTG). After 5 h at 37 °C cells were harvested by centrifugation (5000g, 4 °C, 15 min). The pellets were resuspended in 20 mM Tris, 150 mM NaCl, pH 7.8 (buffer A) supplemented with lysozyme, DNase I and PMSF for the lysis. The lysate was clarified by centrifugation at 12000 g at 4 °C for 1 h and loaded onto a HisTrap HP column (GE Healthcare) equilibrated with 98% buffer A and 2% buffer B (20 mM Tris, 150 mM NaCl, 500 mM imidazole, pH 7.8). The column was washed with 95% buffer A and 5% buffer B and then His-tagged 3CLpro was eluted with a linear gradient of imidazole ranging from 25 mM to 500 mM. Pooled fractions containing target protein was subjected to buffer exchange with buffer A using a HiPrep 26/10 desalting column (GE Healthcare). Next, PreScission protease was added to remove the C-terminal His tag (20 µg of PreScission protease per mg of target protein) at 12 °C overnight. Protein solution was loaded onto a HisTrap HP column connected to a GSTtrap FF column (GE Healthcare) equilibrated in buffer A to remove the GST-tagged PreScission protease, the His-tag, and the uncleaved protein. 3CLpro was finally purified with a Superdex 75 prep-grade 16/60 (GE Healthcare) SEC column equilibrated with buffer C (20 mM Tris, 150 mM NaCl, 1 mM EDTA, 1 mM DTT, pH 7.8). Fractions containing the target protein at high purity were pooled, concentrated at 30 uM and flash-frozen in liquid nitrogen for storage in small aliquots at -80 °C.

### Recombinant 3CLpro characterization and enzymatic activity

The molecular mass of the recombinant SARS-CoV-2 3CLpro was determined by direct infusion electrospray ionization mass spectrometry (ESI-MS) on a Xevo G2-XS QTOF mass spectrometer (Waters). Samples were diluted in 50% acetonitrile with 0.1% of formic acid to achieve a final 1 µM concentration of protein. The detected species displayed a mass of 33796.64 Da, which matches very closely the value of 33796.81 Da calculated from the theoretical full-length protein sequence (residues 1-306). To characterize the enzymatic activity of our recombinant 3CLpro, we adopted a FRET-based assay using the fluorogenic substrate 5-FAM-AVLQ↓SGFRK(DABCYL)K (SEQ ID N0 4 ; Proteogenix) harbouring the cleavage site of SARS-CoV-2 3CLpro (↓ indicates the cleavage site). The fluorescence of the intact peptide is very low since the fluorophore 5-FAM and the quencher Dabcyl are in close proximity. When the substrate is cleaved by the protease, the fluorophore and the quencher are separated, increasing the fluorescence signal. Freshly unfrozen recombinant SARS-CoV-2 3CLpro was used in our assays. The assay was performed by mixing 0.05 µM 3CLpro with different concentrations of substrate (1-128 µM) in the reaction buffer (20 mM Tris-HCI, 100 mM NaCl, 1 mM EDTA and 1 mM DTT, pH 7.3) in the final volume of 100 µL. Fluorescence intensity (Ex = 485 nm/Em = 535 nm) was monitored at 37 °C with a microplate reader VictorIII (Perkin Elmer) for 50 min. A calibration curve was created by measuring multiple concentrations (from 0.001 to 5 µM) of free fluorescein in a final volume of 100 µL reaction buffer. Initial velocities were determined from the linear section of the curve, and the corresponding relative fluorescence units per unit of time (ΔRFU/s) was converted to the amount of the cleaved substrate per unit of time (µM/s) by fitting to the calibration curve of free fluorescein. Inner-filter effect corrections were applied for the kinetic measurements according to refs. The catalytic efficiency kcat/km resulted in 4819± 399 s⁻¹M⁻¹, in line with data of the literature.

### Binding assessment by Nuclear Magnetic Resonance

NMR screening experiments were acquired with a Bruker Neo 600 MHz spectrometer, equipped with nitrogen cooled Prodigy CryoProbe 5 mm at 298 K. The ligand binding was monitored by WaterLOGSY (wLogsy) and Saturation Transfer Difference (STD) experiments in the presence and in the absence of the protein. Samples contained 10 µM of 3CLpro and ligand concentration varying from 100 µM to 2 mM of ligand dissolved in 150 mM NaCl, 20 mM Phosphate, 5% D2O, 4% DMSO-d6 (pH=7.3).

Water-LOGSY experiments were performed with a 180° inversion pulse applied to the water signal at ~4.7 ppm using a Gaussian-shaped selective pulse of 5 ms. Each Water-LOGSY spectrum was acquired with 320 scans a mixing time of 1.5s and a relaxation delay of 4.5 s. STD experiments were acquired with 256 scans. Selective saturation of the protein at 0.4 ppm frequency was carried out by a 2 s pulse train (60 Gaussian pulses of 50 ms separated by 1 ms intervals) included in the relaxation delay and a 30 ms spin-lock was used to reduce the broad background, protein signal. ILOE experiments were achieved with samples having ligands at 2 mM concentration by recording 2D [1H,1H]-NOESY spectra in presence and in absence of the protein (20 µM), with 16 scans for each of 400 indirect points, and mixing times of 600 msec and a recycle delay of 1.5 sec. In the experiments, water suppression was achieved by the excitation sculpting pulse scheme.

### Binding studies by Mass Spectrometry

Samples were prepared by mixing appropriate volumes of 3CLpro (10 µM final) with each compound in the reaction buffer (20 mM Tris-HCI, 100 mM NaCl, 1 mM EDTA and 1 mM DTT, pH 7.3). The final mixtures contained 1:1 or 10:1 of compound:protein molar ratio. Samples were incubated at room temperature for 20 min before analysis. Control experiments were performed on 10 µM solutions of 3CLpro in the absence of compound. Mass spectrometric analyses were carried out in positive ion mode by Electrospray lonisation Mass Spectrometry (ESI-MS) under denaturing conditions *i.e.* 50% acetonitrile with 0.1% formic acid on a mass spectrometer equipped with a Q-Tof analyser (Micro) (Waters, Manchester, UK) or a Q-Tof Xevo G2S (Waters, Manchester, UK). Data were processed by using MassLynx V4.1 software.

### Inhibitory activity of compounds of the invention

The FRET-based assay as detailed above was used to evaluate the ability of the compounds to inhibit its activity *in vitro.* Inhibition of 3CLpro by the tested compounds results in a decrease of the fluorescence signal compared to the 3CLpro activity in the absence of an inhibitor. A preliminary screening was first performed at a single compound concentration to rapidly identify the compounds able to inhibit 3CLpro activity and to rank them according to their inhibitory activity. The protein was diluted in the reaction buffer (20 mM Tris-HCI, 100 mM NaCl, 1 mM EDTA and 1 mM DTT, pH 7.3) and pipetted into a 96-well plate to the final protein concentration of 0.02 µM in 100 µL final volume. Each compound at the final concentration of 100 µM was incubated with 3CLpro for 20 minutes at room temperature. After incubation, the peptide substrate (5 µM final) was added to initiate the reaction which was monitored for 50 min at 37°C. The final DMSO amount was 3,75%. Two controls are prepared for each experiment: the peptide substrate in the absence of 3CLpro (0% 3CLpro activity, hence minimal fluorescence intensity detected) and the reaction mixture in the absence of compound (100% 3CLpro activity, therefore maximal fluorescence intensity detected). Following the preliminary screening, the most active compounds (hits) were tested at increasing concentrations (0.25, 0.5, 1, 5, 25, 50, 100, 150 µM) to determine the dose-response curves and calculate IC50 values fitted by using GraphPad Prism 5 software. Each experiment was performed in triplicate to calculate an average and standard deviation.

### RESULTS

Results are summarized in table III below. All the tested compounds bind the recombinant 3CLpro in NMR experiments and show an inhibitory activity.

### Reversible inhibitors

**Table III**

| **Compounds** | **Binding Activity** |
|---|---|
| 3 or 4 | +++ |
| 5 or 6 | +++ |
| 7 or 8 | ++ |
| 17 or 18 | ++ |
| 1 or 2 | ++ |
| 9 or 10 | +++ |
| 11 or 12 | ++ |
| 15 or 16 | ++ |
| 19 or 20 | ++ |
| 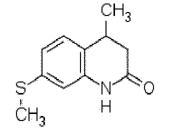 | +/- |
| 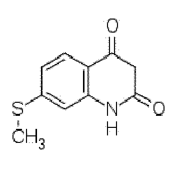 | +/- |
| 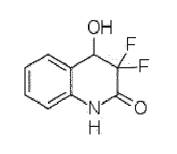 | +/- |
| 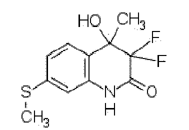 | +/- |

| | |
|---|---|
| +++ : strong binder; ++ : binder; +/- : not to very low binder | |

KD is determined using the data from Saturation Transfer Difference (STD) NMR experiments as exposed above and applying equation 34 in the procedure proposed by Dalvit et al. (2019).

IC50 is determined as described above.

All the tested compounds of the invention of table III are found effective in binding 3CLpro at its substrate-binding pocket. Accordingly, the tested compounds are also found reversible inhibitors of 3CLpro peptidase activity.

### Irreversible inhibitors

**Table IV**

| **Compound** | **IC50** |
|---|---|
| 13 | 2,27 ± 0,05 µM |
| 14 | 0,83 ± 0,05 µM |

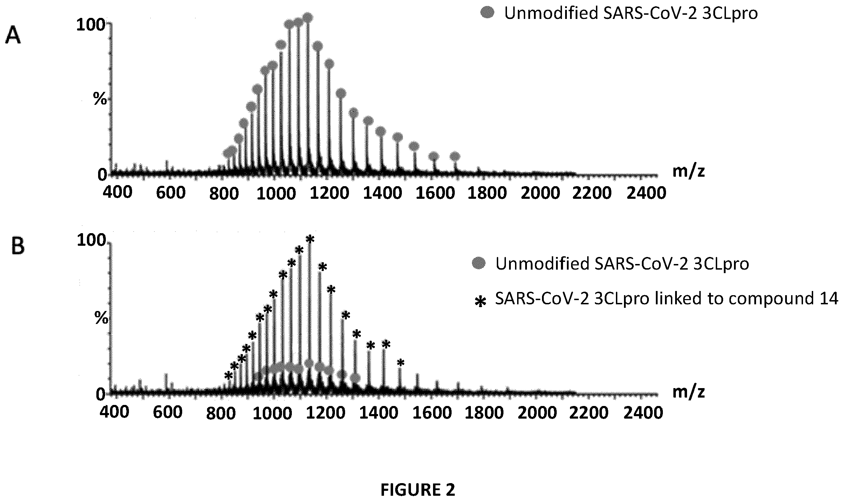

IC(50) is determined as explained above, amongst all the tested irreversible inhibitors compounds 13 and 14 are found particularly effective. Covalent binding is confirmed by ESI mass spectrometry as exposed above and illustrated in Figure 2 for compound 14 (for the state of charge +31 of 3CLpro).

### EXAMPLE 3: Inhibition of spread of SARS-CoV-2 in infected cultured cells.

The protocol which is used is mainly the one described in Rathnayake et al. (2020). Briefly, medium containing DMSO (<0.1%) or the tested compound (up to 100 µM) is added to confluent cells Vero E6 cells inoculated with -50 to 100 PFU per well of SARS-CoV-2, and medium containing various concentrations of each compound and agar is applied to the cells. After 48 to 72 hours, plaques in each well are counted.

Tested compounds of the invention are found effective in reducing the numbers of plaques, thereby showing an impact of said compounds on the spread of virus in cultured cells.

### BIBLIOGRAPHY

Dalvit C, Parent A, Vallée F, Mathieu M, Rak A. Fast NMR Methods for Measuring in the Direct and/or Competition Mode the Dissociation Constants of Chemical Fragments Interacting with a Receptor. ChemMedChem. 2019 Jun 5;14(11):1115-1127. doi: 10.1002/cmdc.201900152.

Mody V, Ho J, Wills S, Mawri A, Lawson L, Ebert MCCJC, Fortin GM, Rayalam S, Taval S. Identification of 3-chymotrypsin like protease (3CLPro) inhibitors as potential anti-SARS-CoV-2 agents. Commun Biol. 2021 Jan 20;4(1):93. doi: 10.1038/s42003-020-01577-x. PMID: 33473151; PMCID: PMC7817688.

Popp_M, Stegemann_M, Metzendorf_M-I, Gould_S, Kranke_P, Meybohm_P, Skoetz_N, Weibel_S. Ivermectin for preventing and treating COVID-19. Cochrane Database of Systematic Reviews 2021, Issue 7. Art. No.: CD015017. DOI: 10.1002/14651858.CD015017.pub2.

Rathnayake AD, Zheng J, Kim Y, Perera KD, Mackin S, Meyerholz DK, Kashipathy MM, Battaile KP, Lovell S, Perlman S, Groutas WC, Chang KO. 3C-like protease inhibitors block coronavirus replication in vitro and i3CLprove survival in MERS-CoV-infected mice. Sci Transl Med. 2020 Aug 19;12(557):eabc5332. doi: 10.1126/scitranslmed.abc5332.

Rosa SGV and Santos WC. Clinical trials on drug repositioning for COVID-19 treatment. Rev Panam Salud Publica. 2020;44:e40.

Stahl P.H, Wermuth C.G.. Pharmaceutical Salts: Properties, Selection, and Use. Wiley, 2011.

Singh_B, Ryan_H, Kredo_T, Chaplin_M, Fietcher_T. Chloroquine or hydroxychloroquine for prevention and treatment of COVID-19. Cochrane Database of Systematic Reviews 2021, Issue 2. Art. No.: CD013587.DOI: 10.1002/14651858.CD013587.pub2

Zhang L, Lin D, Sun X, Curth U, Drosten C, Sauerhering L, Becker S, Rox K, Hilgenfeld R. Crystal structure of SARS-CoV-2 main protease provides a basis for design of i3CLproved α-ketoamide inhibitors. Science. 2020 Apr 24;368(6489):409-412. doi: 10.1126/science.abb3405

## Claims

1. A compound of general formula I : 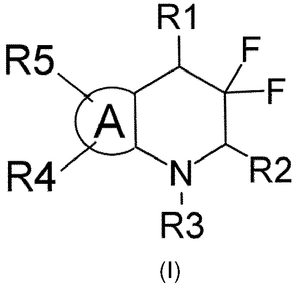 in any enantiomeric, diastereomeric form, or any salt and/or solvate thereof, wherein :
- R₁ and R₂ are each independently from the other selected from : a hydrogen atom, (C₁-C₆) alkyl, OH, OR₆, SR₆ and an oxo group,
- R3 is selected from a hydrogen atom or a (C₁-C₆) alkyl,
- ring A is selected from a 5-membered aromatic ring, a 5-membered heteroaromatic ring, a 6 membered aromatic cycle or a 6 membered heteroaromatic ring,
- R₄ is selected from SO₂R₇, SONHR₇, OR₇, SOR₇, SR₇, (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
- R₅ is selected from H or a halogen atom,
- R₆ is a (C₁-C₆) alkyl, R₇ is selected from (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl,
with the proviso that at least one of R1 or R2 is different from an hydrogen atom.

2. The compound according to claim 2 wherein R₁ and R₂ are each independently from the other selected from a hydrogen atom, OH, (C₁-C₆) alkyl, and an oxo group.

3. The compound according to any one of claims 1 or 2 wherein ring A is selected from a 6 membered aromatic cycle or a 6 membered heteroaromatic ring.

4. The compound according to any one of claims 1-3, wherein R₄ is selected from SR7, (C₁-C₆) alkyl, cycloalkyl, heterocycloalkyl cycloalkyl (C₁-C₆) alkyl, heterocycloalkyl (C₁-C₆) alkyl, aryl, heteroaryl, aryl (C₁-C₆) alkyl, heteroaryl (C₁-C₆) alkyl.

5. The compound according to any one of claims 1-4, wherein said compound is of formula Ia : 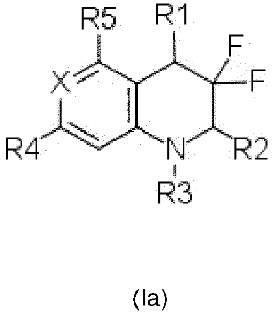 wherein X is an atom selected from C and N, in any enantiomeric, diastereomeric form, or any salt and/or solvate thereof.

6. The compound according to any one of claims 1, 2 or 4, wherein said compound is of formula Ib: 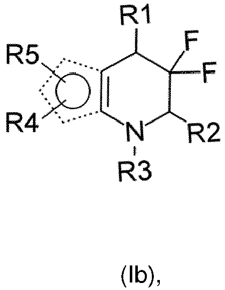 in any enantiomeric, diastereomeric form, or any salt and/or solvate thereof.

7. The compound according to any one of claims 1-6 selected from : 3,3-difluoro-(R)-4-hydroxy-7-(methylsulfanyl)-1,2,3,4-tetrahydroquinolinone ; 3,3-difluoro-(S)-4-hydroxy-7-(methylsulfanyl)-1,2,3,4-tetrahydroquinolinone ; 3,3-difluoro-7-(methylsulfanyl)-1,2,3,4-tetrahydro-1,6-naphthyridin-4-(R)-ol ; 3,3-difluoro-7-(methylsulfanyl)-1,2,3,4-tetrahydro-1,6-naphthyridin-4-(S)-ol S ; 3,3-difluoro-(R)-4-hydroxy-7-(methylsulfanyl)-1,2,3,4-tetrahydroquinolin-2-one ; 3,3-difluoro-(R)-4-hydroxy-7-(methylsulfanyl)-1,2,3,4-tetrahydroquinolin-2-one ; 3,3-difluoro-(R)-4-hydroxy-7-(methylsulfanyl)-1,2,3,4-tetrahydro-1,6-naphthyridin-2-one ; 3,3-difluoro-(S)-4-hydroxy-7-(methylsulfanyl)-1,2,3,4-tetrahydro-1,6-naphthyridin-2-one ; 3,3-difluoro-(S)-4-methyl-7-(methylsulfanyl)-1,2,3,4-tetrahydroquinolin-2-one ; 3,3-difluoro-(R)-4-methyl-7-(methylsulfanyl)-1,2,3,4-tetrahydroquinolin-2-one ; 3,3-difluoro-(S)-4-methyl-7-(methylsulfanyl)-1,2,3,4-tetrahydroquinoline ; 3,3-difluoro-(R)-4-methyl-7-(methylsulfanyl)-1,2,3,4-tetrahydroquinoline ; 3,3-difluoro-7-(methylsulfanyl)-1,2,3,4-tetrahydro-1,6-naphthyridine-2,4-dione ; 3,3-difluoro-7-(methylsulfanyl)-1,2,3,4-tetrahydroquinoline-2,4-dione ; 3,3-difluoro-(R)-4-hydroxy-7-(cyclopropylsulfanyl)-1,2,3,4-tetrahydroquinoline-2-one ; 3,3-difluoro-(S)-4-hydroxy-7-(cyclopropylsulfanyl)-1,2,3,4-tetrahydroquinoline-2-one ; 3,3,5-trifluoro-(R)-4-hydroxy-7-(methylylsulfanyl)-1,2,3,4-tetrahydroquinoline-2-one ; 3,3,5-trifluoro-(S)-4-hydroxy-7-(methylylsulfanyl)-1,2,3,4-tetrahydroquinoline-2-one ; 5,5-difluoro-(R)-4-hydroxy-1,3-dimethyl-1H,4H,5H,6H,7H-pyrazolo[3,4-b]pyridin-6-one ; 5,5-difluoro-(S)-4-hydroxy-1,3-dimethyl-1H,4H,5H,6H,7H-pyrazolo[3,4-b]pyridin-6-one ; 5,5-difluoro-(R)-4-hydroxy-3-methyl-1H,4H,5H,6H,7H-pyrazolo[3,4-b]pyridin-6-one ; 5,5-difluoro-(S)-4-hydroxy-3-methyl-1H,4H,5H,6H,7H-pyrazolo[3,4-b]pyridin-6-one ; 3,5,5-trifluoro-(R)-4-hydroxy-1-methyl-1H,4H,5H,6H,7H-pyrazolo[3,4-b]pyridin-6-one ; 3,5,5-trifluoro-(S)-4-hydroxy-1-methyl-1H,4H,5H,6H,7H-pyrazolo[3,4-b]pyridin-6-one and 5,5-difluoro-1,3-dimethyl-1H,4H,5H,6H,7H-pyrazolo[3,4-b]pyridine-4,6-dione.

8. The compound according to any one of claims 1-7, **characterized in that** it is capable of *in vitro* inhibiting a viral 3-chemotrypsin-like protease (3CLpro), for example a 3CLpro of a coronavirus, for example of MERS-CoV, of a SARS-CoV, for example, a 3CLpro of SARS-CoV1 or a 3CLpro of SARS-CoV-2.

9. The compound according to claim 8, **characterized in that** it is a covalent binder of a viral 3-chymotrypsin-like protease (3CLpro), for example a 3CLpro of a coronavirus, for example of MERS-CoV, a SARS-CoV, for example, a 3CLpro of SARS-CoV1 or a 3CLpro of SARS-CoV-2.

10. The compound according to claim 8, **characterized in that** it is a non-covalent binder of a viral 3-chymotrypsin-like protease (3CLpro), for example a 3CLpro of a coronavirus, for example of MERS-CoV, a SARS-CoV, for example, a 3CLpro of SARS-CoV1 or a 3CLpro of SARS-CoV-2.

11. A pharmaceutical composition comprising a compound according to any one of claims 1-10.

12. A compound according to any one of claims 1-10, for use as a medicament.

13. A compound according to any one of claims 1-10, for use in the prophylaxis, treatment or combination thereof of an infection to a coronavirus in a subject in need thereof.

14. A compound according to any one of claims 1-10, for use in the prophylaxis, treatment or combination thereof of a condition or symptom caused by a coronavirus.

15. The compound for use according to any one of claims 13 or 14 wherein said coronavirus is SARS-CoV-2.
